# EUROPEAN PATENT SPECIFICATION

(11) **EP 4 291 267 B1**
(45) Date of publication and mention of the grant of the patent: **25.11.2026**
(21) Application number: 21840935.7
(22) Date of filing: 21.12.2021
(51) Int. Cl.: A61M 5/24, A61M 5/32

(54) **SYRINGE CARRIERS**
SPRITZENTRÄGER
PORTE-SERINGUES

(30) Priority: 15.02.2021 US 202163149380 P; 25.02.2021 US 202163153408 P; 09.03.2021 US 202163158580 P; 18.03.2021 US 202163162601 P; 23.04.2021 US 202163178577 P; 13.08.2021 EP 21191324
(43) Date of publication of application: 20.12.2023
(73) Proprietor: SHL Medical AG, 6302 Zug (CH)
(72) Inventor: CARLSSON, Daniel, 131 28 Nacka Strand (SE); CHIOU, Meng-Jhen, Taoyuan City, 338 (TW); CHAO, Pei Yu, Taoyuan City, 338 (TW)
(86) International application number: PCT/EP2021/086941
(87) International publication number: WO 2022/171343

(56) References cited:
- EP-A1- 2 601 988
- EP-A1- 3 302 632
- WO-A1-2019/224782
- WO-A1-2021/008839
- GB-A- 2 461 732
- US-A- 5 509 904

## Description

### TECHNICAL FIELD

Syringe carriers for medicament delivery devices such as autoinjectors.

### BACKGROUND

Medicament delivery devices such as autoinjectors often have a syringe carrier to support a syringe (typically a glass syringe) within the medicament delivery device. Although existing syringe carriers can be effective at supporting the syringe, the inventors have appreciated that further improvements can be made. WO2016/193348 describes an audible indicator for a drug delivery device, which drug delivery device includes a drive sub assembly comprising a rear case. WO2019/224782A1 describes a syringe holder to receive forces acting on a syringe assembly and to transmit the forces into the housing, so that stresses acting on a glass body can be avoided or reduced.

### SUMMARY OF THE INVENTION

The invention concerns a number of different concepts for supporting a syringe in a medicament delivery device as described below.

The invention is defined in the independent claim. Further aspects and preferred embodiments are defined in the dependent claims. Any aspects, embodiments and examples of the present disclosure which do not fall under the scope of the appended claims do not form part of the invention and are merely provided for illustrative purposes.

An examplary aspect comprises a method of assembling a sub-assembly of a medicament delivery device, the method comprising the steps of:
providing a housing, a syringe carrier and a syringe, wherein the housing extends in a longitudinal direction along a longitudinal axis from a proximal end to a distal end; inserting the syringe carrier into the housing so that the syringe carrier is aligned with the housing along the longitudinal axis; and inserting the syringe into the syringe carrier in the longitudinal direction, wherein the syringe carrier remains aligned with the longitudinal axis during insertion of the syringe into the syringe carrier, and wherein, as the syringe is inserted into the syringe carrier, the syringe rotates from a first position parallel to the longitudinal axis to a second position that is not parallel to the longitudinal axis and then to a third position parallel to the longitudinal axis.. This can allow the syringe carrier to engage a shoulder of the syringe, and allows for assembly without any deformation, either of the syringe carrier or the syringe.

Optionally, the syringe carrier is inserted into the housing before the syringe is inserted into the syringe carrier. Optionally, the syringe carrier is inserted into the distal end of the housing. Optionally, the syringe is inserted into a distal end of the syringe carrier.

Optionally, the step of inserting the syringe carrier into the housing comprises inserting the syringe carrier into a distal position in the housing, and the syringe carrier is subsequently moved in the proximal direction relative to the housing to a proximal position in the housing.

Optionally, the syringe is moved with the syringe carrier from the distal position to the proximal position. Optionally, when the syringe is in the third position, a shoulder of the syringe abuts a protrusion of the syringe carrier.

An examplary aspect comprises a method of assembling a medicament delivery device comprising the method according to any previous claim.

An aspect comprises a syringe carrier for a medicament delivery device, the syringe carrier comprising a tubular body extending along a longitudinal axis from a proximal end to a distal end, wherein at least part of the tubular body is a helical spring. This can allow for a dampening support of the syringe - the flexing of the spring can help damp impact forces, such as the force of dropping a medicament delivery device comprising the syringe carrier. This can also allow for insertion of a syringe into a syringe carrier without any permanent deformation of the syringe or syringe carrier.

The tubular body comprises a distal part and a proximal part, wherein the proximal part is a helical spring. The distal part is a tubular base.

Optionally, the distal part is attached to the proximal part by an arm extending in the direction of the longitudinal axis.

The helical spring extends from a distal end to a proximal end relative to the longitudinal axis, and the helical spring is tapered, with a larger internal diameter at the distal end of the helical spring than at the proximal end of the helical spring. Optionally, the helical spring is frustoconical or cylindrical.

Optionally, the helical spring is an integral part of the tubular body.

Optionally, the helical spring comprises a rib or protrusion extending inwardly from the proximal end of the helical spring. This can support a shoulder of a syringe.

Another examplary aspect concerns a medicament delivery device comprising a syringe carrier as described above. Optionally, the medicament delivery device is an autoinjector.

Another examplary aspect concerns a medicament delivery device comprising any of the syringe carriers described above. Optionally, the medicament delivery device is an autoinjector.

### BRIEF DESCRIPTION OF THE DRAWINGS

Embodiments of the present disclosure will now be described by way of example only and with reference to the accompanying drawings as listed below.
Figures 1 to 3 show perspective views of a syringe carrier.
Figures 4 and 5 show partial cross-section views during assembly of an autoinjector comprising the syringe carrier of Figure 1.
Figure 6 shows a partial cross-section view of an autoinjector comprising the syringe carrier of Figure 1.
Figures 7 to 10 show different partial cross-section and cross-section views of an autoinjector comprising the syringe carrier of Figure 1.
Figure 11 shows a perspective view of another syringe carrier with a syringe for context.
Figures 12 to 14 show perspective views of another syringe carrier.
Figures 15 to 17 show perspective views of another syringe carrier.
Figure 18 shows a perspective view of part of the syringe carrier of Figure 15.
Figures 19 and 20 show perspective views of another syringe carrier.
Figure 21 shows a cross-section view of a syringe carrier similar to the one in Figure 19, along with a syringe in the syringe carrier.
Figures 22 and 23 show perspective views of an alternative distal structure for the syringe carriers of Figures 19 to 21, with a syringe also shown in the syringe carrier in Figure 23.
Figure 24 shows a perspective view of an example autoinjector.
Figure 25 shows a perspective view of an example syringe.
Figure 26 shows a cross-section view of the example syringe of Figure 25.

### DETAILED DESCRIPTION

Embodiments of the present disclosure will now be described by way of example only and with reference to the accompanying drawings. The invention is defined by the appended claims, to which reference should now be made.

In general, the present disclosure describes various syringe carriers for medicament delivery devices such as autoinjectors. Generally, these syringe carriers are for use in devices in which the syringe is inserted into the device (for example into a housing of the device) in the axial direction during assembly.

An example focusses on the idea of a syringe carrier with partial axial support.

The 1 ml tall syringe is by far the most used primary container within the auto injector market. The syringe usually is made out of glass; a material less suited for impact loads. The 1 ml tall syringe can be fitted with a flexible needle shield only or with an additional rigid needle shield. The latter protects the user from needle stick injuries when administered as a prefilled syringe only, but creates a problem when assembled into an auto injector. When the diameter of the RNS is equally large as the barrel diameter of the 1 ml tall syringe, this limits the possibility of supporting the syringe at its neck or shoulder. Supporting the syringe by the finger flange may be undesirable since the flange is more sensitive to breakage due to its geometrical shape.

This problem is usually solved by having a c-shaped support that is allowed to bend during syringe assembly or by flexing supports that are later supported by the auto injector enclosure. Both of the mentioned solutions require the plastic to bend as the syringe is inserted. The bending motion caused by the diameter change as the RNS enters naturally creates forces on the RNS during assembly. This can cause the RNS to move which in turn can affect the sterility of the primary container or cause the gap between the RNS and syringe neck to close. The latter can prevent the supports on the syringe carrier from engaging fully and can cause jamming during assembly - resulting in a useless or broken final product.

Considering the potential problems with the existing designs, the applicant has appreciated that an alternative approach could provide a more satisfactory solution.

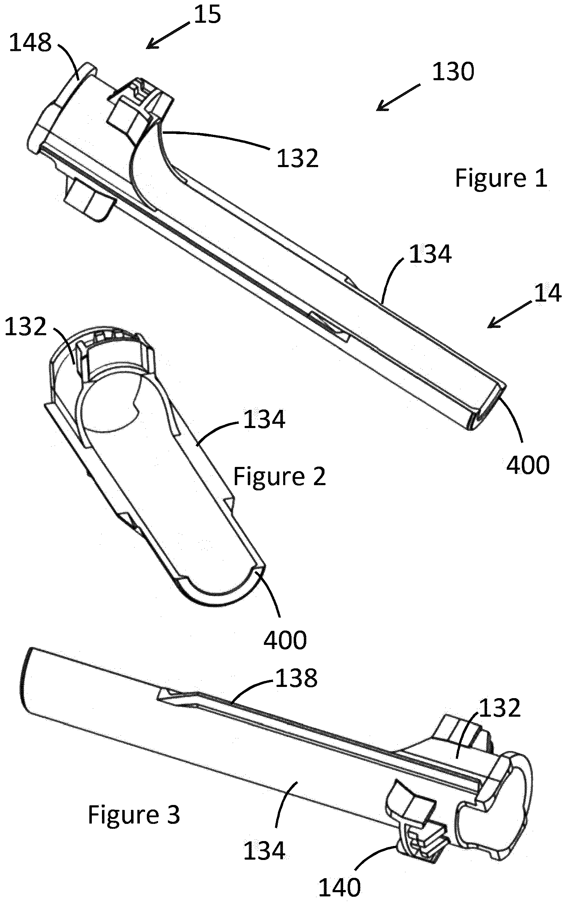

A syringe carrier with a partial axial support is suggested to allow the syringe to be inserted without any plastic deformation or large forces applied to the RNS during assembly. Figures 1 to 3 show an example syringe carrier 130 from various angles. The syringe carrier 130 has a partial support intended to fit in between the neck of the syringe and the RNS. The support runs less than 180 degrees around the neck and the syringe is simply allowed to tilt (rotate) about 1 degree during assembly to allow for the RNS to pass the supports (for example between 0.5 and 1.5 degrees). However, designs with a different level of rotation could also be used, depending for example on the amount of spare space inside the housing for the syringe to rotate.

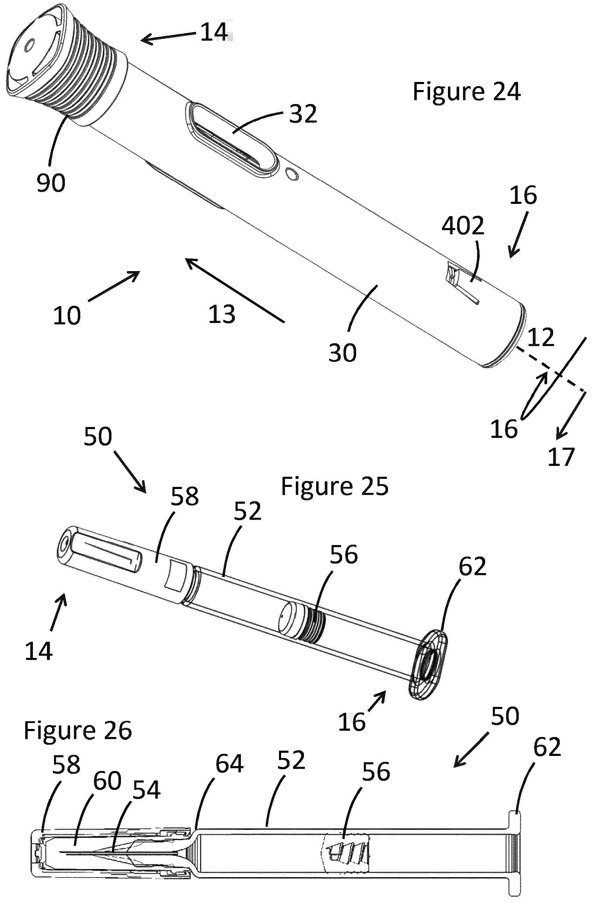

The syringe carrier 130 extends from a proximal end 14 to a distal end 15. The syringe carrier comprises a base 132 and an arm 134, with the arm extending in the proximal direction from the base 132. At the proximal end of the arm (although it could also be spaced apart in the longitudinal direction from the proximal end of the arm), an inwardly extending protrusion 400 is provided. This protrusion is configured to enter the gap between a syringe shoulder 64 and the rigid needle shield 58 of the syringe (see Figure 26, for example). In this example, the proximally facing side of the protrusion extends perpendicular to the longitudinal axis, and the distally facing side of the protrusion is angled towards the longitudinal axis (which can help support the shoulder of the syringe by mirroring the shape of the shoulder of the syringe). However, these particular shapes for the proximally facing side and the distally facing side of the protrusion 40 are optional and could be varied, depending for example on the shape of other medicament delivery device components (including the shape of the syringe).

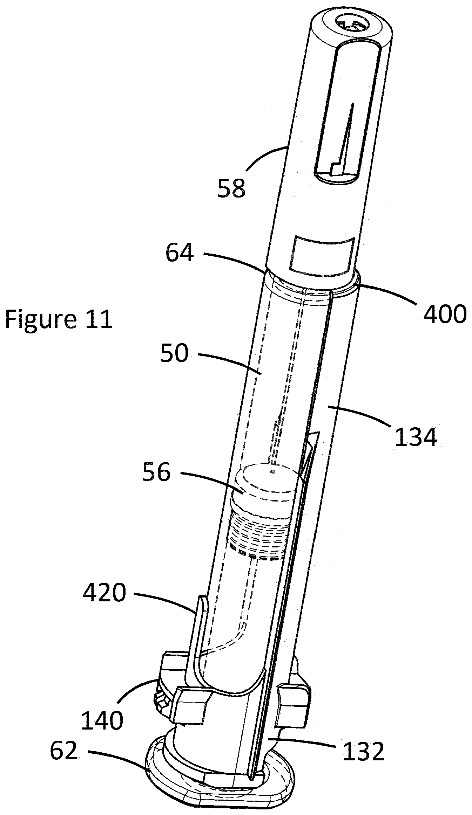

In this case, a single protrusion 400 is provided (in this case a rib), though multiple protrusions could alternatively be provided. In this case, the protrusion 400 extends the full width of the arm in the circumferential direction, though this is also optional. Although only one arm is provided, two or more arms could alternatively be provided (see Figure 11 for an example).

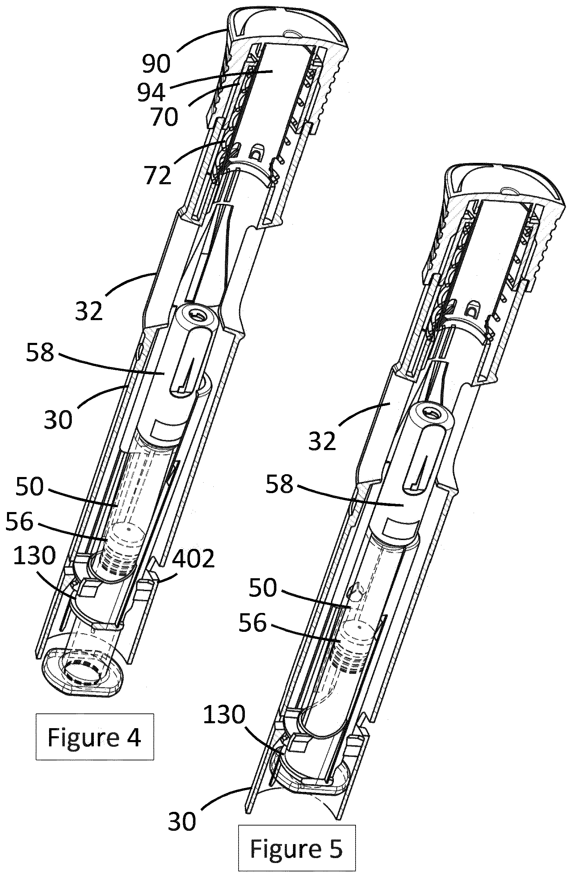

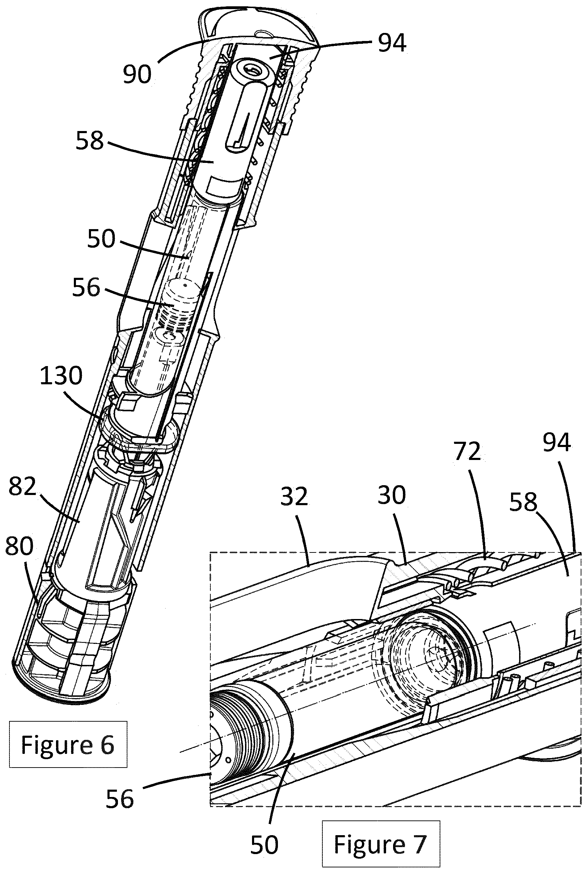

Figures 4 to 6 show an example of medicament delivery device assembly using a syringe carrier as described above. Although various details of the medicament delivery device (which in this example is an autoinjector) are shown, the key components in this assembly method are the housing, the syringe carrier and the syringe, and the other components are not essential. The particular shapes of the housing, syringe carrier and syringe could also be varied (for example as discussed elsewhere herein), and also do not need to have this particular structure. In this example, the medicament delivery device comprises a cap 90, an RNS remover 94 in the cap, an optional needle guard 70, and an optional needle guard spring 72, along with the housing 30 (which comprises an optional window 32), the syringe carrier 130 and the syringe 50. As can be seen in Figure 6, the medicament delivery device comprises a powerpack, of which a rear housing 80 and a rotator 82 are visible. In this example a spring-driven powerpack is envisioned, with a plunger rod and a spring not visible in the Figures. Alternative powerpacks, for example gas-powered or electrically driven powerpacks, could be used instead.

The method of assembly will now be described. In general, the idea is that a sub-assembly of a medicament delivery device is assembled by firstly providing a housing, a syringe carrier and a syringe. The syringe carrier is then inserted into the housing and the syringe is inserted into the syringe (typically, though not necessarily, in that order). The syringe is inserted into the syringe carrier in the longitudinal direction with the syringe and the syringe carrier aligned along the longitudinal axis. During the insertion of the syringe into the syringe carrier, the syringe rotates relative to the longitudinal axis to allow a rigid needle shield of the syringe to pass a protrusion 400 of the syringe carrier.

The method of assembly will now be described in more detail with reference to the Figures. Firstly, various components such as the cap (including the RNS remover) and the needle guard (including the needle guard spring) would typically be attached to the housing before the method that will now be described, although these could also be attached to the housing at a different stage in the assembly process as well. In this example, the syringe carrier is first inserted into the housing, with the insertion carried out by inserting the syringe carrier into the distal end of the housing and moving the syringe carrier in the proximal direction relative to the housing (although the syringe could alternatively be inserted into the syringe carrier before the syringe is inserted into the housing). When the syringe carrier is inserted into the housing, the syringe carrier is inserted to a first position (a distal position). In this position, the syringe carrier is attached to the housing by engaging protrusions 140 of the syringe carrier with corresponding flexible arms 402 on the housing (though the flexible arms could be on the syringe carrier and the protrusions on the housing instead). An example of a flexible arm 402 is most clearly visible in Figure 24, and would typically include an inwardly extending protrusion to engage the protrusion 140 of the syringe carrier.

When in the first position, the syringe carrier is typically aligned with the longitudinal axis of the housing (and remains aligned with the longitudinal axis during subsequent steps). In this example, this means that the base of the syringe carrier, which is tubular, is coaxial with the housing. While the syringe carrier is in the first position, the syringe is inserted into the syringe carrier. The syringe is inserted into the distal end of the syringe carrier and moved in the proximal direction relative to the syringe carrier. In this example, as the syringe carrier is already in the housing, the syringe is therefore also inserted into the housing in this step (though this is not essential, for example if the syringe carrier is partially or entirely outside of the housing when the syringe is inserted into the syringe carrier).

As the syringe enters the syringe carrier, the RNS 58 of the syringe will encounter the protrusion 400 of the syringe carrier. In this particular example, the combination of the base 132 and the protrusion 400 should naturally result in the syringe carrier rotating to allow the RNS 58 to pass the protrusion 400 (though this rotation could also just be carried out as part of the insertion step without relying on the protrusion to cause the rotation). The syringe rotates from a position (orientation) parallel to the longitudinal axis to a position not parallel to the longitudinal axis. Once the RNS 58 has passed the protrusion 400, the syringe can rotate back (again either naturally, for example due to gravity, or by appropriate manipulation of the syringe during assembly). The syringe therefore starts off lined up along the longitudinal axis (i.e. parallel to the longitudinal axis)(first syringe position), then is rotated so that it is at an angle to the axis (i.e. not parallel to the longitudinal axis)(second syringe position)(for example up to 20 degrees, such as 0.5 to 10 degrees, 0.5 to 1.5 degrees or 2 to 5 degrees), and then is rotated back in line with the axis (i.e. parallel with the longitudinal axis)(third position). In the third syringe position (which is the final position, at least relative to the syringe carrier), the protrusion 400 of the syringe carrier is in the gap between the RNS 58 and the shoulder 64, which allows the protrusion 400 to support the shoulder 64 of the syringe. Typically, the shoulder abuts the protrusion. This completes assembly of the sub-assembly.

In the first and third positions, the syringe and the syringe carrier are typically coaxial. In the first and third positions, the syringe and the housing are typically coaxial.

In this example, the syringe carrier and the syringe are then moved together in the proximal direction relative to the housing from the first position (distal position)(as shown in Figure 5) to a second position (proximal position) as shown in Figure 6. Alternatively, the syringe carrier is inserted directly into its final position, rather than initially being arranged in a distal position and then subsequently moving to a proximal position. However, providing a distal position and a proximal position can make assembly easier, for example by making it easier to insert the syringe into the syringe carrier and/or by inserting the syringe into the syringe carrier in a position where there is space for the syringe to rotate - this can allow the syringe to be better supported in the final position, as there is no need for space for the syringe to rotate during assembly.

A powerpack (including the rear housing 80 and the rotator 82 in this example) is then attached to the distal end of the housing 30. This would typically result in a completed medicament delivery device, although further steps such as the addition of labels could subsequently be carried out if not already completed at an earlier stage of medicament delivery device assembly.

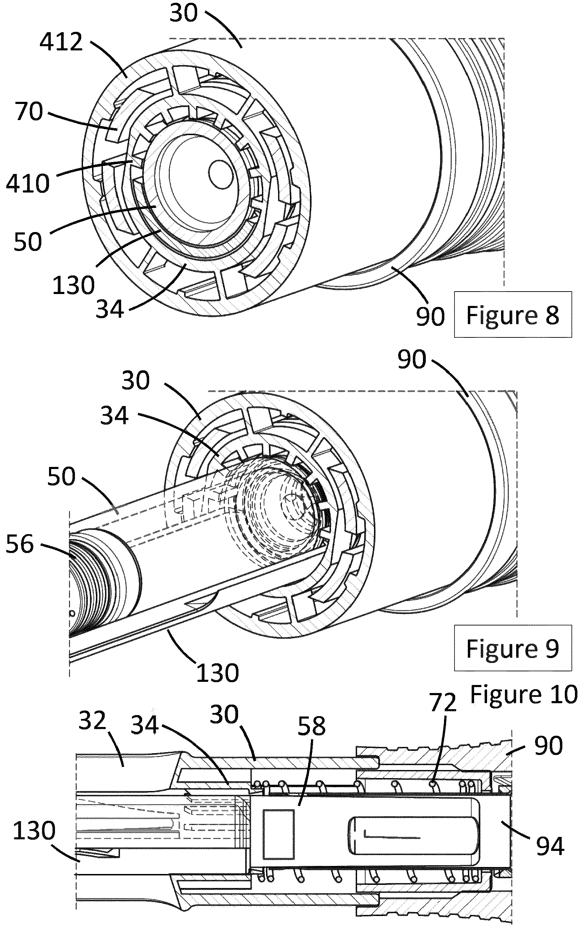

Some further details of the particular example shown in the Figures will now be described, although these features are also not essential for the general method being described herein. Figures 7 to 10 in particular show greater detail of the area of the medicament delivery device around the syringe carrier. In Figure 8, multiple ribs 410 with an inner diameter close to (i.e. the same size or slightly larger than, for example less than 2mm larger) the outer diameter of the syringe interact with the syringe barrel to prevent any radial movement of the syringe. This is possible in this particular example because the syringe is inserted into the syringe carrier while the syringe carrier is in a distal position, with the proximal position as shown here being the final position for the syringe and syringe carrier relative to the housing. The ribs are attached to an inner part 34 of the housing 30, with the inner part 34 of the housing 30 attached to an outer part 412 of the housing 30. The syringe carrier is also restricted from moving in the radial direction by the inner part 34 of the housing 30. Movement relative to the housing in the longitudinal direction can be restricted by other components and/or by attachment points such as snap-fits between the syringe carrier (for example the protrusions 140 of the syringe carrier) and the housing or another component of the medicament delivery device. Figures 9 and 10 show different views of the syringe and syringe carrier fitted in the enclosure body after final assembly.

Figure 11 shows an alternative example in which the syringe carrier additionally comprises a second arm 420 extending along the syringe (i.e. extending in the direction of the longitudinal axis) to help orient the syringe once the RNS has passed the syringe carrier support. The syringe carrier is otherwise the same as the syringe carrier described above (Figures 1 to 3). The second arm 420 is opposite the arm 134 relative to the longitudinal axis. The second arm can help support the syringe, particularly during assembly (particularly if the syringe is inserted into the syringe carrier before the syringe carrier is partially or fully in the housing), but does not unduly restrict rotation of the syringe as it is much shorter (in the longitudinal direction) than the arm 134. The arm 134 could also be flexible. In this example, the second arm is less than 25% of the length of the arm. One second arm is provided in this example, although multiple second arms could be provided.

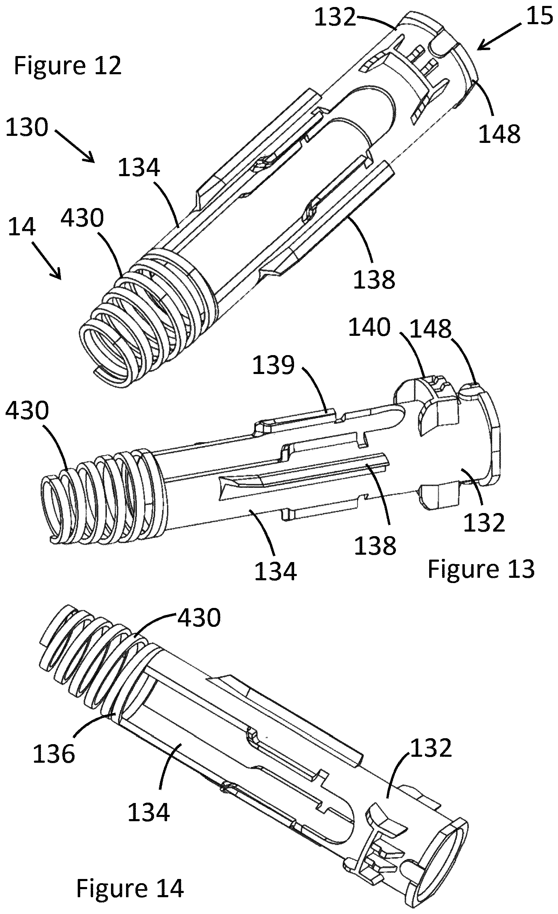

Another example concerns a syringe carrier with a helical structure (Figures 12 to 18). The helical structure is flexible, like a spring, and will herein be called a helical spring 430. The diameter of the opening in the proximal end of the syringe carrier is smaller than the diameter of the opening in the distal end of the syringe carrier. During assembly, the syringe is inserted into the distal end of the syringe carrier and pushed towards the proximal end relative to the syringe carrier. When the RNS of the syringe passes through the proximal end of the syringe carrier, the syringe carrier flexes, with the opening in the proximal end of the syringe carrier increasing in diameter. The proximal end of the syringe carrier can then flex back into the gap between the medicament holder and the syringe shoulder, and can therefore support the syringe and restrict movement of the syringe in the proximal direction relative to the syringe carrier and therefore also other parts of an assembled medicament delivery device. Movement of the syringe in the distal direction relative to the syringe carrier can be restricted by other components of a completed medicament delivery device.

An example of a syringe carrier with a helical spring 430 is shown in Figures 12 to 14. The syringe carrier 130 is typically tubular, and extends from a proximal end 14 to a distal end 15. A distal part of the syringe carrier is a base 132, which in this example is tubular. two arms 134 extend in the proximal direction from the proximal end of the base. A proximal part of the syringe carrier is the helical spring 430. The helical spring 430 is attached to the proximal ends of the two arms 134. In this example, the helical spring 430 is attached to a ring 136, which is in turn attached to the two arms 134. However, the ring 136 is optional, and the helical spring 430 could alternatively be attached directly to the arms 134. The arms are also optional, and the helical spring could be attached directly to the base.

The helical spring has a larger diameter at the distal end than at the proximal end, with the diameter decreasing in the proximal direction (i.e. a tapered helical spring). This is particularly relevant for the inner diameter (internal diameter) of the helical spring (and is optional for the outer diameter of the helical spring, though the outer diameter also decreases in this example). When a syringe such as the syringe 50 shown in Figure 25 is inserted into the distal end of the syringe carrier 130 of Figure 12, the RNS 58 of the syringe will enter into the helical spring 430. The helical spring is sized to support the RNS. As a result, the outer diameter of the RNS (at least at the distal end of the RNS) is larger than the inner diameter of the proximal end of the helical spring. When the RNS is pushed into the helical spring, it will therefore push the proximal end of the helical spring outwards, increasing the inner diameter of the proximal end of the helical spring (the pushing of the RNS will typically also cause the helical spring to flex and therefore increase in length in the longitudinal direction as well).

Once the RNS has passed the proximal end of the helical spring, the helical spring will flex back again, with the inner diameter of the proximal end of the helical spring decreasing again. As a result, the proximal end of the helical spring will be in the gap between the RNS 58 and the shoulder 64 of the syringe, and can thereby support the shoulder of the syringe. Optionally, another feature of a completed medicament delivery device, such as part of a housing (such as a rear housing) or of a needle guard, could limit or stop the proximal end of the helical spring from flexing away from the longitudinal axis in a completed medicament delivery device, which can increase the support for the syringe.

The helical spring is flexible. As a result, the helical spring is compressible in the longitudinal direction. The helical spring can also flex in the plane perpendicular to the longitudinal axis, which is particularly relevant for the proximal end of the helical spring, as it allows the RNS to pass the proximal end of the helical spring during assembly.

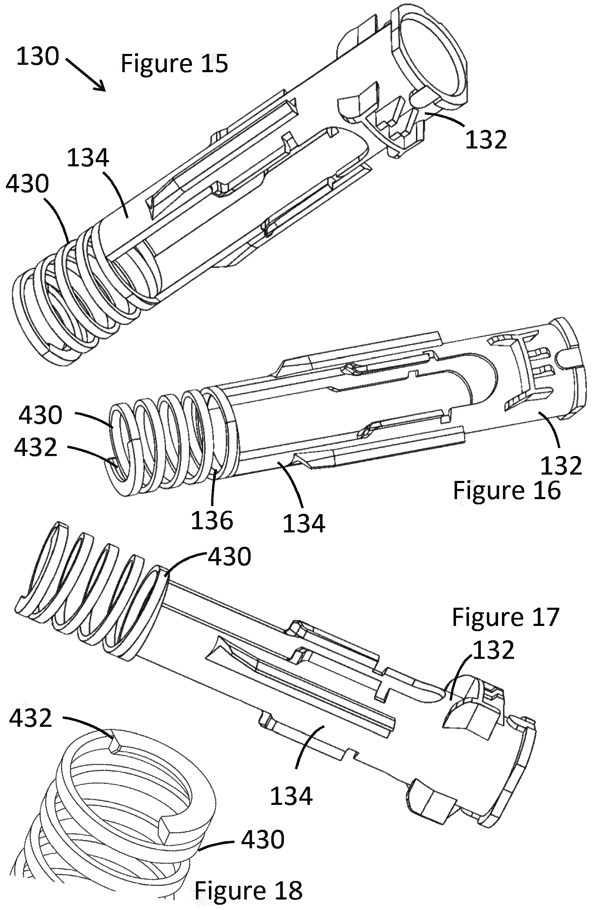

A second example syringe carrier is shown in Figures 15 to 18. This syringe carrier is similar to the one in Figures 12 to 14, and will not be described again in detail. The difference between the syringe carrier shown in Figures 15 to 18 and the syringe carrier shown in Figures 12 to 14 is that the diameter of the syringe carrier remains the same along the length of the helical spring in the longitudinal direction (i.e. the helical spring is cylindrical rather than frustoconical as in Figure 12). Other helical spring shapes could also be used, depending for example on the shape of a corresponding housing of a medicament delivery device.

The syringe carrier in Figures 15 to 18 comprises an optional rib 432 extending towards the longitudinal axis from the proximal end of the helical spring 430 (alternatively, the proximal end of the helical spring is configured to directly support a shoulder of a syringe). A rib like this (or alternatively one or more protrusions) could optionally also be provided on the syringe carrier in Figures 12 to 14. The rib can help support a shoulder of a syringe, and could be shaped to match the shape of the shoulder of a syringe (as is the case in this example, as can be seen in Figure 18 - the surface of the rib is angled relative to the longitudinal direction to match the angle of a shoulder of a syringe). In the example in Figure 15, the inner diameter of the proximal end of the helical spring needs to be smaller than the inner diameter of the ring 136, as the ring cannot expand to let the RNS pass during assembly. This is achieved in this particular example (Figure 15) by providing a rib 432 extending towards the longitudinal axis from the proximal end of the helical spring - as a result, the inner diameter of the helical spring is narrower than the inner diameter of the ring, and an RNS can therefore pass through the ring and then push the helical spring (so that the inner diameter of the helical spring increases so that the RNS can pass the rib 432). The rib can then move back into the gap between the RNS and the shoulder of the syringe after the RNS has passed the helical spring. Alternatively, the ring 136 could be removed. As a result, the entire helical spring could expand (i.e. the inner diameter could increase) to allow an RNS to pass during assembly. As the arms 134 can be flexible, the proximal ends of the arms 134 could also move apart from one another if needed.

Optionally, the helical spring could be spaced apart from the proximal end, rather than being the proximal-most feature of the syringe carrier. Optionally, the thickness of the spring could be varied to vary flexibility (and therefore also compressibility) at different parts of the spring. Depending on the shape of other medicament delivery device components, the base 132 may be optional.

In a method of assembling a medicament delivery device, a syringe and a syringe carrier with a helical spring as described herein are provided. The syringe is attached to the syringe carrier, and the syringe and syringe carrier are inserted into a housing. Optionally, the syringe is attached to the syringe carrier before the syringe is inserted into the housing. Optionally, the syringe carrier is attached to the housing before the syringe is inserted into the syringe carrier.

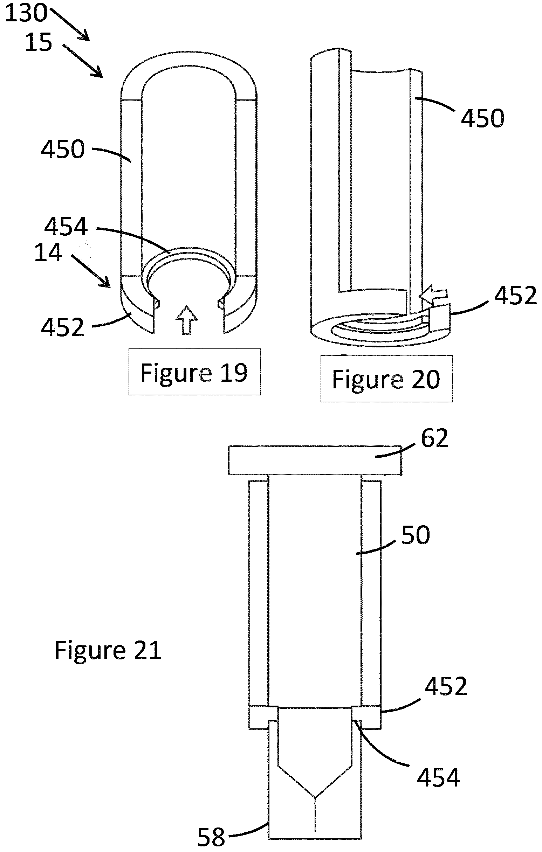

In another example, the syringe carrier is a part of a cylinder, along with a c-clip at the proximal end, as shown in Figures 19 and 20. During assembly, the syringe can be inserted radially or longitudinally into the syringe carrier. The position of the syringe and syringe carrier after assembly is shown in Figure 21. The syringe carrier of Figure 21 is a slightly different design to that in Figures 19 and 20 (the rib is in a slightly different location compared to the example in Figure 19), although the basic concept is the same. The syringe and syringe carrier can subsequently be inserted into a housing of a medicament delivery device (alternatively, the syringe carrier could be inserted into a housing of a medicament delivery device before the syringe carrier is inserted into the housing).

As shown in Figure 19, the syringe carrier comprises a partial tube section 450 (in this case half a cylinder, though the particular shape could be varied), with a c-clip 452 at the proximal end. The c-clip extends further in the circumferential direction than the partial tube section. On the inside surface of the c-clip, a rib 454 extends towards the longitudinal axis. The rib 454 can help support a shoulder of a syringe. The c-clip is flexible so that the c-clip can flex away from the longitudinal axis to allow an RNS of a syringe to pass the rib and to then flex back into a gap between the shoulder and the RNS.

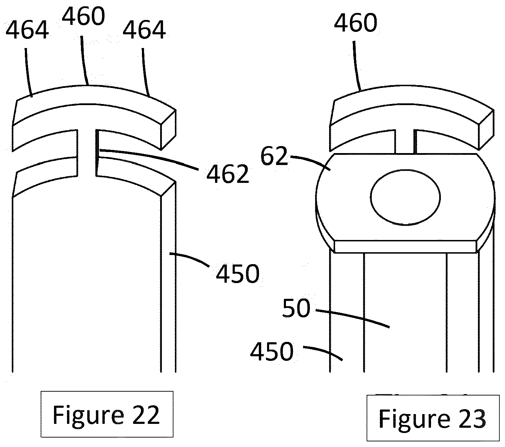

The syringe carrier can also optionally support the syringe at the flange 62 of the syringe 50 as well. The c-clip can support the proximal end of the syringe at the shoulder (in the gap between the RNS and the shoulder). In the example in Figures 19 to 21, the flange of the syringe is beyond the distal end of the syringe carrier, although the distal end of the syringe carrier could additionally or alternatively support the flange. In another alternative example, as shown in Figures 22 and 23, a structure can extend from the distal end of the syringe carrier to hold the flange and thereby help support the syringe - in this example, the structure is a T-shaped protrusion 460. The T-shaped protrusion comprises a longitudinally extending strut 462 extending from the distal end of the partial tube section 450. At the distal end of the longitudinally extending strut 462, two circumferentially extending arms 464 are arranged, each extending around the longitudinal axis in opposite directions from the longitudinally extending strut 462. The flange 62 of the syringe 50 fits between the circumferentially extending arms 464 and the distal end of the partial tube section 450. In this example, the T-shaped protrusion 460 is an integral part of the syringe carrier 130, but the structure could optionally be provided only to support the flange temporarily during assembly (the housing of the medicament delivery device could subsequently help support the syringe instead, for example). Part or all of the T-shaped protrusion 460 could be made of a flexible material such as a thermoplastic elastomer (TPE) to cushion the flange of the syringe.

A rib that extends towards the axis (see Figures 19 and 20) can be provided on the c-clip; this can help the c-clip support the syringe. Alternatively, one or more protrusions can be provided instead of a rib.

A partial tube section that is half a tube is shown in Figure 19, although this could be varied - in Figure 19, the partial tube section extends half way (so 180 degrees) in the circumferential direction, and this could be increased or decreased - for example, Figure 22 shows a partial tube section that extends less than 180 degrees in the circumferential direction.

A number of modifications can be made to the examples in Figures 19 to 23. Optionally, the housing of the medicament delivery device is tubular and comprises one or more protrusions extending towards the axis from an inner surface of the housing. The one or more protrusions can help support the syringe. Optionally, the syringe carrier comprises a base (such as a tubular base, like the base shown in Figure 12), and the distal end of the base can support the flange of a syringe. Optionally, the syringe carrier comprises a protrusion extending from an outer surface of the syringe carrier (i.e. away from the longitudinal axis); such a protrusion can engage with a corresponding feature on another medicament delivery device component (such as a housing or a needle guard) to help align the syringe carrier correctly during assembly. Similarly, instead of a protrusion, a slot could be provided on the outer surface of the syringe carrier, along with a protrusion on another medicament delivery device component (such as a housing or a needle guard), to help align the syringe carrier correctly during assembly.

The examples herein focus on autoinjectors, but the examples described herein could be implemented in other medicament delivery devices more generally, such as in pen injectors. Some of the examples herein focus on 1 ml syringes, but the designs described herein could also be used on other volumes and other types of medicament container, for example a syringe without an attached needle rather than a syringe with an attached needle. An example of an autoinjector 10 that could comprise the syringe carriers described herein is shown in Figure 24. The example autoinjector extends along an axis 12 in an axial direction 13 between a distal end 15 and a proximal end 14, with a radial direction 17 and a circumferential direction 16 also depicted for reference. A housing (or body) 30 and a cap 90 of the autoinjector can be seen, along with an optional window 32 in the body. The autoinjector can house a syringe. The autoinjector typically includes features such as a powerpack and a needle guard inside the housing. The shape of the housing and of the cap could be varied from those shown in the example - for example, the housing could be triangular in cross section perpendicular to the axis rather than circular, could be an irregularly-shaped tube rather than a cylinder, and/or the housing could be two or more components rather than a single component. The autoinjector shown does not have an activation button, though one could be provided (i.e. a three-step autoinjector rather than a two-step autoinjector). An optional arm 402, which could be used to support other components of the autoinjector during or after assembly, is also shown.

Figures 25 and 26 show an example of a syringe for reference. This particular syringe 50 comprises a medicament holder (medicament container) 52, a needle 54, a stopper 56, a rigid needle shield (RNS) 58, a flexible needle shield (FNS) 60, a flange 62 and a shoulder 64. The syringe extends from a proximal end 14 to a distal end 15. The medicament holder 52 is tubular (specifically cylindrical in this example), with the flange 62 at the distal end of the medicament holder 52 and the needle 54 at the proximal end of the medicament holder 52. The stopper 56 is in the medicament holder 52. The flexible needle shield 60 extends around the needle 54, and the rigid needle shield 58 extends around the flexible needle shield. The shoulder 64 is the proximal end of the medicament holder 52. One particular example syringe is described here, but other syringes could be used. For example, a needle 54 is included in examples described herein, but other medicament delivery members such as jet injectors could alternatively be used, or the needle could be provided separately rather than as an integral part of the syringe. A needle shield comprising a rigid needle shield 58 and a flexible needle shield 60 is included in examples described herein, but the examples described herein could be used with needle shields without a flexible needle shield or even entirely without a needle shield, although the examples described herein can be particularly beneficial when used with syringes with an RNS. The flange 62 is also typically optional. The syringe could be various sizes, including but not limited to 1ml and 2.25ml.

Example mechanical powerpacks are described herein (for example the powerpack shown in Figure 6), but other types of powerpack could be used instead, for example an electrically powered powerpack or a gas-powered powerpack. An example of a device in which syringe carriers as described herein could be used is provided in WO2011/123024.

A base 132 is described herein. Typically, the base is depicted as the distal portion of the syringe carrier, but could alternatively be spaced apart in the axial direction from the distal end of the syringe carrier.

Arms 134 are described herein. Most (though not all) of the examples herein use two arms, although in the examples with two arms, one, three or more arms could alternatively be provided. Similarly, other features that are provided (arms, protrusions, cut-outs, recesses and the like) can generally be provided in a quantity different to the specific number described in the examples given.

Many of the syringe carriers described herein comprise some kind of optional protrusion or rib. For example, a number of the examples, including the syringe carriers shown in Figures 12 and 15, comprise two ribs 138, four second ribs 139 and two protrusions 140. The ribs 138 extend in the longitudinal direction, with the proximal end of each rib 138 attached to an arm 134 and the distal end of each rib 138 attached to the base 132. The second ribs 139 extend in the longitudinal direction. A second rib 139 is arranged on each side of each arm 134 in the circumferential direction. The protrusions 140 are attached to the base 132. Another optional feature is a distal flange 148, which is also present in many of the syringe carriers described herein. The flange 148 is attached to the base 132 and extends in the radial direction away from the axis and extends in the circumferential direction around the base. These ribs, protrusions and flanges can provide various advantages, including helping to align the syringe carrier relative to other features during assembly, maintaining rigidity of the syringe carrier, and/or fixing the position of the syringe carrier relative to other components of a medicament delivery device in a completed device. Whilst these protrusions 140, ribs 138, 139 and flanges 148 are depicted as having a particular shape, these shapes could be varied depending on factors such as the desired rigidity and on the shape of other components within a particular design of medicament delivery device.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component. The circumferential direction describes a direction extending around the axis, so around the circumference of a device or component, and the radial direction extends perpendicular to the axis.

Similarly, the terms "transverse", "transversal" and "transversally" refer to a direction generally perpendicular to the longitudinal direction.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

Various modifications to the embodiments described are possible and will occur to those skilled in the art without departing from the invention which is defined by the following claims.

## Claims

1. A syringe carrier (130) for a medicament delivery device, the syringe carrier (130) comprising a tubular body extending along a longitudinal axis from a proximal end to a distal end,
wherein the tubular body comprises a distal part and a proximal part, wherein the distal part is a tubular base (132)
**characterized in that**
the proximal part is a helical spring (430),
wherein the helical spring (430) extends from a distal end to a proximal end relative to the longitudinal axis, and the helical spring (430) is tapered, with a larger internal diameter at the distal end of the helical spring (430) than at the proximal end of the helical spring (430).

2. The syringe carrier (130) of claim 1, wherein the distal part of the tubular body is attached to the proximal part of the tubular body by an arm (134) extending in the direction of the longitudinal axis.

3. The syringe carrier of claim 1 or claim 2, wherein the helical spring (430) is frustoconical.

4. The syringe carrier of any of claims 1 to 3, wherein the helical spring (430) comprises a rib or protrusion extending inwardly from the proximal end of the helical spring (430).

5. A medicament delivery device comprising the syringe carrier (130) of any of claims 1 to 4.

## Patentansprüche

1. Spritzenträger (130) für eine Medikamentenabgabevorrichtung, der Spritzenträger (130) umfassend einen rohrförmigen Körper, der sich entlang einer Längsachse von einem proximalen Ende zu einem distalen Ende erstreckt,
wobei der rohrförmige Körper einen distalen Teil und einen proximalen Teil umfasst,
wobei der distale Teil eine rohrförmige Basis (132) ist,
**dadurch gekennzeichnet, dass**
der proximale Teil eine Schraubenfeder (430) ist,
wobei sich die Schraubenfeder (430) von einem distalen Ende zu einem proximalen Ende relativ zu der Längsachse erstreckt und die Schraubenfeder (430) verjüngt ist, wobei sie einen größeren Innendurchmesser an dem distalen Ende der Schraubenfeder (430) als an dem proximalen Ende der Schraubenfeder (430) aufweist.

2. Spritzenträger (130) nach Anspruch 1, wobei der distale Teil des rohrförmigen Körpers an dem proximalen Teil des rohrförmigen Körpers durch einen Arm (134) befestigt ist, der sich in der Richtung der Längsachse erstreckt.

3. Spritzenträger nach Anspruch 1 oder 2, wobei die Schraubenfeder (430) kegelstumpfförmig ist.

4. Spritzenträger nach einem der Ansprüche 1 bis 3, wobei die Schraubenfeder (430) eine Rippe oder einen Vorsprung umfasst, die/der sich von dem proximalen Ende der Schraubenfeder (430) nach innen erstreckt.

5. Medikamentenabgabevorrichtung, umfassend den Spritzenträger (130) nach einem der Ansprüche 1 bis 4.

## Revendications

1. Support de seringue (130) pour un dispositif d'administration de médicament, le support de seringue (130) comprenant un corps tubulaire s'étendant le long d'un axe longitudinal d'une extrémité proximale à une extrémité distale,
dans lequel le corps tubulaire comprend une partie distale et une partie proximale,
dans lequel la partie distale est une base tubulaire (132)
**caractérisée en ce que**
la partie proximale est un ressort hélicoïdal (430),
dans lequel le ressort hélicoïdal (430) s'étend d'une extrémité distale à une extrémité proximale par rapport à l'axe longitudinal, et le ressort hélicoïdal (430) est effilé, avec un diamètre interne plus grand à l'extrémité distale du ressort hélicoïdal (430) qu'à l'extrémité proximale du ressort hélicoïdal (430).

2. Support de seringue (130) selon la revendication 1, dans lequel la partie distale du corps tubulaire est fixée à la partie proximale du corps tubulaire par un bras (134) s'étendant dans la direction de l'axe longitudinal.

3. Support de seringue selon la revendication 1 ou la revendication 2, dans lequel le ressort hélicoïdal (430) est tronconique.

4. Support de seringue selon l'une quelconque des revendications 1 à 3, dans lequel le ressort hélicoïdal (430) comprend une nervure ou une saillie s'étendant vers l'intérieur à partir de l'extrémité proximale du ressort hélicoïdal (430).

5. Dispositif d'administration de médicaments comprenant le support de seringue (130) selon l'une quelconque des revendications 1 à 4.
